# EUROPEAN PATENT APPLICATION

(11) **EP 4 734 090 A1**
(43) Date of publication of application: **29.04.2026**
(21) Application number: 25203684.3
(22) Date of filing: 22.09.2025
(51) Int. Cl.: G08B 29/04, G08B 29/14, G08B 17/107

(54) **EVENT MONITORING AND DETECTION DEVICE TESTING**

(30) Priority: 15.10.2024 US 202418916100
(71) Applicant: Honeywell International Inc., Charlotte, NC 28202 (US)
(72) Inventor: WOLF, Benjamin H., Charlotte, 28202 (US); DEARDEN, Christopher, Charlotte, 28202 (US); BARSON, Michael, Charlotte, 28202 (US)
(74) Representative: Haseltine Lake Kempner LLP

(57) **Abstract**

Devices, methods, and systems for event monitoring and detection device (100, 200, 300, 400) testing are described herein. One device includes a memory (124, 524) and a processor (126, 526) to execute instructions stored in the memory to modify a sensitivity level of a sensor (412) of the event monitoring and detection device from a first sensitivity level to a second sensitivity level, cause an air movement device (116, 216, 416) to pass air through a testing chamber (104, 204, 404) of the event monitoring and detection device, cause the sensor to take an environmental particulate matter reading of particle sizes of particulates in the air passing through the testing chamber, and transmit the environmental particulate matter reading to a remote computing device (426).

## Description

### Technical Field

The present disclosure relates generally to devices, methods, and systems for event monitoring and detection device testing.

### Background

Large facilities (e.g., buildings), such as commercial facilities, office buildings, hospitals, and the like, may have an alarm system that can be triggered during an emergency situation (e.g., a fire) to warn occupants to evacuate. For example, an alarm system may include a control panel and a plurality of event monitoring and detection devices located throughout the facility (e.g., on different floors and/or in different rooms of the facility) that can sense an event occurring in the facility and provide a notification of the event to the occupants of the facility via alarms.

Maintaining the alarm system can include regular cleaning and testing of event monitoring and detection devices. Such cleaning and/or testing of event monitoring and detection devices may be mandated by codes of practice in an attempt to ensure that the event monitoring and detection devices are functioning properly.

### Brief Description of the Drawings

Figure 1 illustrates a block diagram of an event monitoring and detection device in accordance with one or more embodiments of the present disclosure.
Figure 2 illustrates a portion of an example of an event monitoring and detection device in accordance with one or more embodiments of the present disclosure.
Figure 3 illustrates a block diagram of an alarm system in accordance with one or more embodiments of the present disclosure.
Figure 4 illustrates a system for event monitoring and detection device testing in accordance with an embodiment of the present disclosure.
Figure 5 is an example of a controller for event monitoring and detection device testing, in accordance with one or more embodiments of the present disclosure.

### Detailed Description

Devices, methods, and systems for event monitoring and detection device testing are described herein. One device includes a memory and a processor to execute instructions stored in the memory to modify a sensitivity level of a sensor of the event monitoring and detection device from a first sensitivity level to a second sensitivity level, cause an air movement device to pass air through a testing chamber of the event monitoring and detection device, cause the sensor to take an environmental particulate matter reading of particle sizes of particulates in the air passing through the testing chamber, and transmit the environmental particulate matter reading to a remote computing device.

As mentioned above, maintaining the alarm system can include regular cleaning and testing of event monitoring and detection devices. However, since tests may only be completed periodically, there is a risk that faulty event monitoring and detection devices may not be discovered quickly or that tests will not be carried out on all the event monitoring and detection devices in an alarm system.

Testing each event monitoring and detection device can be time consuming, expensive, and disruptive to a business. For example, a maintenance engineer is often required to access event monitoring and detection devices which are situated in areas occupied by building users or parts of buildings that are often difficult to access (e.g., elevator shafts, high ceilings, ceiling voids, etc.). As such, the maintenance engineer may take several days and several visits to complete testing of the event monitoring and detection devices, particularly at a large site. Additionally, it is often the case that some event monitoring and detection devices never get tested because of access issues.

In order to ensure event monitoring and detection devices are tested, the event monitoring and detection devices can utilize a self-test procedure. The self-test procedure can be an automatic procedure performed by an event monitoring and detection device without a user, such as a maintenance engineer or other type of user. The self-test procedure can therefore allow event monitoring and detection devices to be tested, even if such event monitoring and detection devices are remotely located and/or difficult to access.

The self-test procedure can include generating a test medium and providing the test medium to a test chamber for sensing. The test medium can be provided from a self-test module included in the event monitoring and detection device. However, if the testing chamber is blocked, the self-test will not be able to be completed. Additionally, if the testing chamber is blocked, normal operation of the event monitoring and detection device may be affected, as the event monitoring and detection device may not be able to monitor for events that occur in the facility.

Additionally, sensing for particulate matter by an event monitoring and detection device in a facility can be an important factor to monitor in order to provide a safe facility for occupants. For example, long-term exposure to particulate matter, such as fine particulate matter (e.g., particles that are 2.5 micrometers or less in diameter, referred to herein as PM2.5 particulate matter) can cause health issues such as heart or lung diseases. While specific sensors exist to measure this particulate matter, such sensors require a high-power budget and specific wiring back to a building management system, which can be a complex and expensive process.

Event monitoring and detection device testing according to the disclosure can allow for the event monitoring and detection device to determine whether the testing chamber is blocked and also measure for particulate matter by utilizing an air movement device included in the self-test module. The self-test can ensure that the testing chamber is not blocked so that the event monitoring and detection device can operate normally to detect events in the facility, as well as measure for particulate matter in the facility without having to utilize specialized sensors and expensive and complex wiring. Additionally, determining whether the testing chamber is blocked can be performed while the event monitoring and detection device is in a normal operational mode listening for events. Accordingly, event monitoring and detection device testing can provide for a more effective, robust, and cost-conscious solution for self-test and particulate monitoring, as compared with previous approaches.

In the following detailed description, reference is made to the accompanying drawings that form a part hereof. The drawings show by way of illustration how one or more embodiments of the disclosure may be practiced.

These embodiments are described in sufficient detail to enable those of ordinary skill in the art to practice one or more embodiments of this disclosure. It is to be understood that other embodiments may be utilized and that mechanical, electrical, and/or process changes may be made without departing from the scope of the present disclosure.

As will be appreciated, elements shown in the various embodiments herein can be added, exchanged, combined, and/or eliminated so as to provide a number of additional embodiments of the present disclosure. The proportion and the relative scale of the elements provided in the figures are intended to illustrate the embodiments of the present disclosure and should not be taken in a limiting sense.

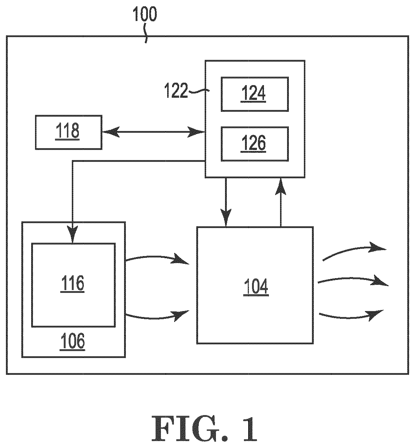

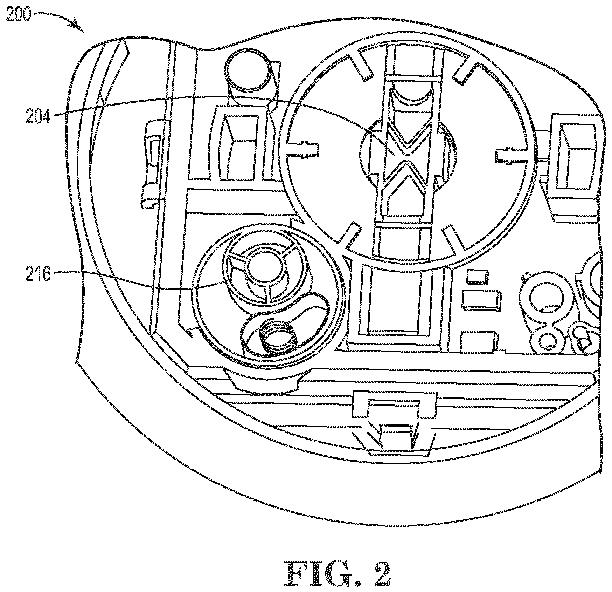

The figures herein follow a numbering convention in which the first digit or digits correspond to the drawing figure number and the remaining digits identify an element or component in the drawing. Similar elements or components between different figures may be identified by the use of similar digits. For example, 104 may reference element "04" in Figure 1, and a similar element may be referenced as 204 in Figure 2.

As used herein, "a", "an", or "a number of" something can refer to one or more such things, while "a plurality of" something can refer to more than one such things. For example, "a number of components" can refer to one or more components, while "a plurality of components" can refer to more than one component.

Figure 1 illustrates a block diagram of an event monitoring and detection device 100 in accordance with one or more embodiments of the present disclosure. The event monitoring and detection device 100 can include a controller (e.g., microcontroller) 122, a sounder 118, a testing chamber 104, a self-test module 106, and an air movement device 116.

The controller 122 can include a memory 124 and a processor 126. Memory 124 can be any type of storage medium that can be accessed by processor 126 to perform various examples of the present disclosure. For example, memory 124 can be a non-transitory computer readable medium having computer readable instructions (e.g., computer program instructions) stored thereon that are executable by processor 126 for event monitoring and detection device testing in accordance with the present disclosure. For instance, processor 126 can execute the executable instructions stored in memory 124 to modify a sensitivity level of a sensor, cause an air movement device to pass air through a testing chamber 104, cause the sensor to take an environmental particulate matter reading, and transmit the environmental particulate matter reading to a remote computing device.

Figure 2 illustrates a portion of an example of an event monitoring and detection device 200 in accordance with one or more embodiments of the present disclosure. The event monitoring and detection device 200 can correspond to the event monitoring and detection device 100 of Figure 1 and can be, but is not limited to, a fire and/or smoke detector of a fire control system.

An event monitoring and detection device 200 can sense an event, such as a fire, occurring in a facility and trigger a response to provide a notification of the event to occupants of the facility. An event response can include visual and/or audio alarms, for example. An event response can also notify emergency services (e.g., fire departments, police departments, etc.). In some examples, a plurality of event monitoring and detection devices can be located throughout a facility (e.g., on different floors and/or in different rooms of the facility).

As shown in Figure 2, the event monitoring and detection device 200 can include a testing chamber 204 and an air movement device 216. The testing chamber 204 can be, for instance, an optical scatter chamber and the air movement device 216 can correspond to the air movement device 116 of Figure 1.

The air movement device 216 can control the airflow through the event monitoring and detection device 200, including the testing chamber 204. For example, the air movement device 216 can move particles, gases, and/or aerosol from a first end of the event monitoring and detection device 200 to a second end of the event monitoring and detection device 200. The air movement device 216 can start responsive to a command and can stop responsive to a command and/or after a particular period of time.

An event monitoring and detection device 200 can automatically or upon command perform a self-test procedure. The self-test procedure can include causing a test medium to be generated, initiating a release of the test medium into the testing chamber 204, and causing a sensor to take a reading of the test medium.

The reading can include measuring a value associated with the test medium in the testing chamber 204. The controller can further compare the value associated with the test medium to a threshold value.

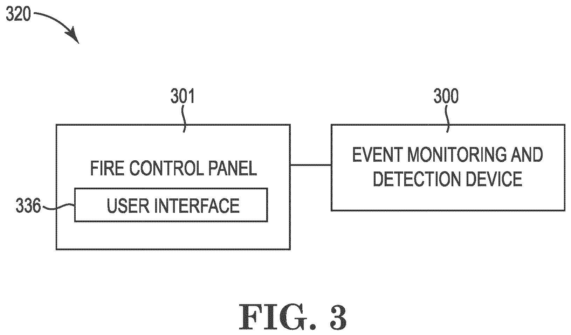

Figure 3 illustrates a block diagram of an alarm system 320 in accordance with one or more embodiments of the present disclosure. The alarm system 320 can include an event monitoring and detection device 300 and a fire control panel 301. The event monitoring and detection device 300 can be, for example, event monitoring and detection device 100 and/or 200 previously described in connection with Figures 1 and 2, respectively.

The fire control panel 301 can be a monitoring device, a fire detection control system, and/or a cloud computing device of the alarm system 320. The fire control panel 301 can be configured to send commands to and/or receive reports from an event monitoring and detection device 300 via a wired or wireless network. For example, the event monitoring and detection device 300 can report a sensor reading during a self-test procedure of the event monitoring and detection device 300. Additionally, in some examples the event monitoring and detection device 300 can report a confirmed event to the fire control panel 301 responsive to a measured value after a particular period of time being greater than a threshold value.

The fire control panel 301 can receive reports from a number of event monitoring and detection devices analogous to event monitoring and detection device 300. For example, the fire control panel 301 can receive reports from each of a number of event monitoring and detection devices analogous to event monitoring and detection device 300 and transmit commands based on the reports from each of the number of event monitoring and detection devices.

In a number of embodiments, the fire control panel 301 can include a user interface 336. The user interface 336 can be a GUI that can provide and/or receive information to and/or from a user and/or the event monitoring and detection device 300. The user interface 336 can display messages and/or data received from the event monitoring and detection device 300. For example, the user interface 336 can alert a user to an unconfirmed event, a confirmed event, and/or a false alarm reported by the event monitoring and detection device 300.

The networks described herein can be a network relationship through which event monitoring and detection device 300 and/or fire control panel 301 can communicate with each other. Examples of such a network relationship can include a distributed computing environment (e.g., a cloud computing environment), a wide area network (WAN) such as the Internet, a local area network (LAN), a personal area network (PAN), a campus area network (CAN), or metropolitan area network (MAN), among other types of network relationships. For instance, the network can include a number of servers that receive information from and transmit information to event monitoring and detection device 300 and/or fire control panel 301 via a wired or wireless network.

As used herein, a "network" can provide a communication system that directly or indirectly links two or more computers and/or peripheral devices and allows a fire control panel to access data and/or resources on an event monitoring and detection device 300 and vice versa. A network can allow users to share resources on their own systems with other network users and to access information on centrally located systems or on systems that are located at remote locations. For example, a network can tie a number of computing devices together to form a distributed control network (e.g., cloud).

A network may provide connections to the Internet and/or to the networks of other entities (e.g., organizations, institutions, etc.). Users may interact with network-enabled software applications to make a network request, such as to get data. Applications may also communicate with network management software, which can interact with network hardware to transmit information between devices on the network.

In some examples, the network can be used by the event monitoring and detection device 300 and/or the fire control panel 301 to communicate with a remote computing device. The remote computing device can be a personal laptop computer, a desktop computer, a mobile device such as a smart phone, a tablet, a wrist-worn device, and/or redundant combinations thereof, among other types of computing devices. The remote computing device can receive reports from a number of event monitoring and detection devices analogous to event monitoring and detection device 300 and/or a number of fire control panels analogous to fire control panel 301 and transmit commands based on the reports to one or more of the number of event monitoring and detection devices and/or one or more of the number of fire control panels.

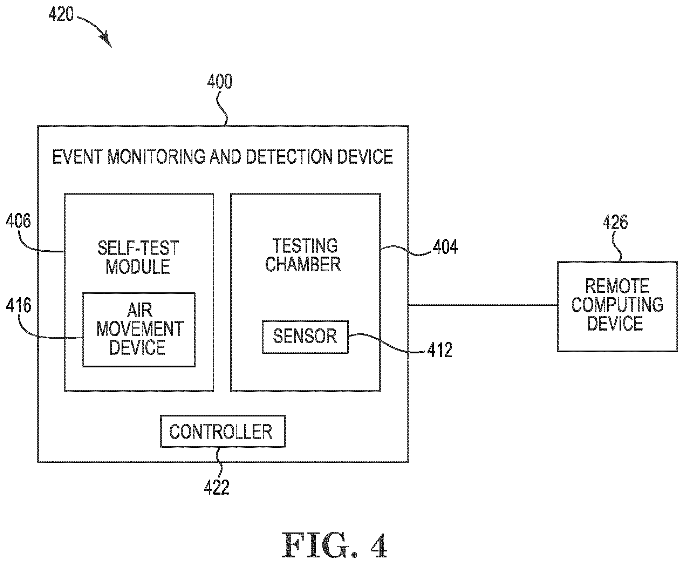

Figure 4 illustrates a system 410 for event monitoring and detection device testing in accordance with an embodiment of the present disclosure. The system 410 can include an event monitoring and detection device 400 and a remote computing device 426.

As mentioned above, an event monitoring and detection device 400 can perform a self-test procedure. The self-test procedure can be a test to ensure the event monitoring and detection device 400 accurately detects events occurring in the area in which the event monitoring and detection device 400 is located. As one example, the self-test procedure can allow for the event monitoring and detection device 400 to detect smoke particulates in the area the event monitoring and detection device 400 is located in order to detect a fire. As part of the self-test procedure, the event monitoring and detection device 400 can take an environmental particulate matter reading during normal operation of the event monitoring and detection device 400. Additionally, the event monitoring and detection device 400 can determine whether the testing chamber 404 of the event monitoring and detection device 400 is blocked utilizing anti-mask testing procedures during normal operation of the event monitoring and detection device 400, as are further described herein.

As illustrated in Figure 4, the event monitoring and detection device 400 can include a self-test module 406, a testing chamber 404, and a controller 422. The self-test module 406 can include an air movement device 416 and the testing chamber 404 can include a sensor 412. The sensor 412 can be, for example, a heat sensor, a smoke sensor, a particulate matter sensor, and/or any other kind of sensor or combination thereof.

The sensor 412 can include a sensitivity level that is variable. As used herein, the term "sensitivity level" refers to an input parameter change that is required to produce a standardized output change. For example, the sensor 412 can have a first sensitivity level and a second sensitivity level, where the second sensitivity level is more sensitive than the first sensitivity level, although the sensor 412 is not limited to just two sensitivity levels. For instance, the sensor 412 can include more than two sensitivity levels.

As mentioned above, the testing chamber 404 can be an optical scatter chamber. The optical scatter chamber can include a light transmitter (e.g., at least one light emitting diode (LED)) and a photosensitive light receiver that can measure a value associated with a test medium located in the testing chamber 404. For example, the testing chamber 404 can pulse the light transmitter to measure whether any particulate matter (e.g., smoke, PM2.5, etc.) is present in the testing chamber 404, as is further described herein.

As mentioned above, the event monitoring and detection device 400 can cause a sensor to take an environmental particulate matter reading for the environment surrounding the event monitoring and detection device 400. Such a reading can take place during a normal operational mode of the event monitoring and detection device 400. For example, during normal operational mode of the event monitoring and detection device 400, the environmental particulate matter reading can be taken while the event monitoring and detection device 400 is further listening for other events (e.g., fire detection). That is, the event monitoring and detection device 400 does not need to be transitioned to a maintenance mode where the event monitoring and detection device 400 does not listen for events.

However, prior to taking the environmental particulate matter reading, the event monitoring and detection device 400 can perform a preliminary event check to determine whether an event is occurring near the event monitoring and detection device 400. For example, the event monitoring and detection device 400 can perform a preliminary event check of smoke in the testing chamber 404 prior to modifying a sensitivity level of the sensor 412, as is further described herein. The preliminary event check can be utilized prior to performing the environmental particulate matter test to ensure there are no actual events (e.g., a fire event) occurring in the facility prior to the environmental particulate matter test.

In order to perform the preliminary event check, the controller 422 can include causing the air movement device 416 to move ambient air through the testing chamber 404. For example, the air movement device 416 (e.g., a fan) can be initiated causing air movement through the testing chamber 404. During the air movement, the controller 422 can cause the sensor 412 to take a preliminary sample of the ambient air passing through the testing chamber 404 to determine whether there are any smoke particles in the ambient air passing through the testing chamber 404. The sensor 412 can take the preliminary sample while at the first sensitivity level and the controller 422 can determine, based on the preliminary sample, whether an event such as a fire is occurring near the event monitoring and detection device 400. For example, if the preliminary sample exceeds an alarm threshold, the event monitoring and detection device 400 can transmit a signal indicating an event (e.g., a fire) is occurring. However, if the preliminary sample does not exceed an alarm threshold, the controller 422 can determine no event is occurring. Therefore, the event monitoring and detection device 400 can perform an environmental particulate matter reading knowing no event is currently occurring, as is further described herein.

In response to the sensor 412 not detecting an event during the preliminary event check (e.g., indicating no real events are occurring in the facility), the controller 422 can initiate the environmental particulate matter test process. For example, the controller 422 can modify a sensitivity level of the sensor 412 from a first sensitivity level to a second sensitivity level. The first sensitivity level can be, for instance, a normal operating sensitivity level for the sensor 412 in order to detect an event during normal operating conditions. The first sensitivity level of the sensor 412 can be, for instance, 0.2 percent obscuration per meter (%OPM).

The controller 422 can modify the sensitivity level by increasing the sensitivity level of the sensor 412 from the first sensitivity level to a second sensitivity level. The second sensitivity level can be more sensitive than the first sensitivity level. For example, the controller 422 can increase the sensitivity level of the sensor 412 from 0.2 %OPM to 20 %OPM (e.g., the second sensitivity level).

Once the sensitivity level of the sensor 412 is increased, the controller 422 can cause the air movement device 416 to pass air through the testing chamber 404. For example, the air movement device 416 can cause air located proximate to the event monitoring and detection device 400 to move into and through the testing chamber 404.

As the air is passing through the testing chamber 404, the controller 422 can cause the sensor 412 to take an environmental particulate matter reading of particle sizes of particulates in the air passing through the testing chamber 404. The sensor 412 can take the environmental particulate matter reading by pulsing a light transmitter, such as an LED, and capturing the light pulses by a photosensitive light receiver, such as a photo diode. The captured light pulses can be utilized to estimate particle sizes of the particulates and quantities of the particulates based on distributions of light scattering intensities from individual particles in the air moving through the testing chamber 404. The distributions of light scattering intensities can be included as data in the environmental particulate matter reading. For example, the environmental particulate matter reading can include a sample for particulate matter in the air passing through the testing chamber 404 that is smaller than an event threshold size. One example of particulate matter the sensor 412 can sense while at the second sensitivity level is PM2.5 particulate matter, although embodiments are not limited to PM2.5 particulate matter. For instance, the sensor 412 can sense particulate matter that is larger or smaller than PM2.5 particulate matter.

The controller 422 can cause the environmental particulate matter reading to be transmitted to a remote computing device 426. The remote computing device 426 can determine, based on the environmental particulate matter reading, whether particulate matter that is less than a threshold size is present in the air passing through the testing chamber 404. For example, the remote computing device 426 can utilize the data in the environmental particulate matter reading to estimate particle sizes from the distribution of light scattering intensity data captured by the sensor 412. In response to the remote computing device 426 determining particulate matter that is less than a threshold size is present, the remote computing device 426 can generate and transmit a notification.

Following the environmental particulate matter test, the controller 422 can again modify the sensitivity level of the sensor 412. For example, the controller 422 can modify the sensitivity level of the sensor 412 by reducing the sensitivity level from the second sensitivity level to the first sensitivity level. At the first sensitivity level, the event monitoring and detection device 400 can go back to sampling around the event monitoring and detection device 400 for events.

The above testing procedure for environmental particulate matter readings can be repeated according to a particular frequency. For example, the above testing procedure can be performed every 5 minutes, every 20 minutes, every hour, etc. Additionally, the particular frequency may be modified based on previous environmental particulate matter readings. For example, the particular frequency may be increased if a previous environmental particulate matter reading indicates particulate matter that is smaller than an event threshold level is detected, and the particular frequency may be decreased if a previous environmental particulate matter reading indicates particulate matter that is smaller than an event threshold level is not detected.

Additionally, in some examples, the above testing procedure for environmental particulate matter readings and/or the anti-mask testing (as is further described herein) can be performed in response to an input. For example, in response to a user input (e.g., to the remote computing device 426, to a mobile device not illustrated in Figure 4, to a control panel, etc.), the above testing procedure for environmental particulate matter readings and/or the anti-mask testing can be performed. Such an approach can allow for "on-demand" testing of the event monitoring and detection device 400.

Accordingly, utilizing the above testing procedure, the event monitoring and detection device 400 can be utilized to detect particulate matter that is smaller than an event threshold size. When this fine particulate matter is detected, a notification can be generated to alert a user that there is an air quality issue detected by the event monitoring and detection device 400.

As mentioned above, the event monitoring and detection device 400 can additionally determine whether the testing chamber 404 is blocked via an anti-mask test, as is further described herein.

In order to perform the anti-mask test, the controller 422 can cause the self-test module 406 to generate an amount of test medium for the testing chamber 404. In some examples, the test medium can be an aerosol. For example, the controller 422 can cause a coil to heat wax until a temperature at which the wax emits an aerosol comprised of smoke particles. The coil and the wax can be located in a self-test module 406 of the event monitoring and detection device 400.

In order to effectively perform the anti-mask test, the amount of test medium has to be less than an alarm threshold of the event monitoring and detection device 400. For example, in order to perform the anti-mask test during a normal operational mode of the event monitoring and detection device 400, the amount of test medium generated can be less than the alarm threshold of the event monitoring and detection device 400 in order to prevent the event monitoring and detection device 400 from transmitting an alarm to, for example, a fire control panel.

Accordingly, the controller 422 can cause the self-test module 406 to generate the amount of test medium for a predetermined amount of time such that the amount of test medium generated is less than the alarm threshold while the sensor 412 is at the second sensitivity level. Generation of the test medium for only the predetermined amount of time can prevent the amount of test medium from exceeding the alarm threshold and causing the event monitoring and detection device 400 to transmit a false alarm.

The controller 422 can cause the air movement device 416 to move the generated test medium into the testing chamber 404 and then evacuate the test medium from the test chamber. For example, the air movement device 416 can cause the test medium to move into the testing chamber 404 and then out of the testing chamber 404. Based on the air movement device 416 evacuating the test medium from the testing chamber 404, the controller 422 can determine whether the testing chamber 404 is blocked.

The obscuration test can include causing, by the controller 422, the sensor 412 to take an initial value (e.g., a clean air value) prior to the test medium being generated and moved into the testing chamber 404. The initial value can be a reference value for the anti-mask obscuration test, as is further described herein. For instance, the initial value can be 0 percent obscuration per meter (%OPM).

After the test medium is generated and the air movement device 416 causes the test medium to move into the testing chamber 404, the sensor 412 can cause the sensor 412 to take a number of obscuration test values. For instance, at a first time the sensor 412 can take a first obscuration test value of 15 %OPM, at a second time the sensor 412 can take a second obscuration test value of 8 %OPM, at a third time the sensor 412 can take a third obscuration test value of 1 %OPM, etc.

The controller 422 can determine that, at the third time, the third obscuration test value of 1 %OPM is within a threshold amount from the initial value (e.g., 0 %OPM). For example, the threshold amount can be 2 %OPM, and as the third obscuration test value of 1 %OPM is within the threshold amount from the initial value, the controller 422 can determine that the third obscuration test value is within the threshold.

The obscuration test can include determining an amount of time taken to evacuate the test medium from the testing chamber 404. If a large amount of time is taken to evacuate the test medium from the testing chamber 404, this may indicate the testing chamber 404 is blocked, whereas if a small amount of time is taken to evacuate the test medium from the testing chamber 404, this may indicate the testing chamber 404 is not blocked, as is further described herein.

Accordingly, the controller 422 can determine an amount of time elapsed between the initial value of 0 %OPM and the obscuration test value that was within the threshold amount of the initial value (e.g., the third obscuration test value). For example, the controller 422 can determine that the amount of time elapsed between the initial value of 0 %OPM and the third obscuration test value of 1 %OPM was 20 seconds.

The controller 422 can compare the amount of time elapsed between the initial value and the obscuration test value that was within the threshold amount of the initial value to a threshold time. In one example, the threshold time can be 30 seconds. The controller can compare the amount of time elapsed (e.g., 20 seconds) to the threshold time (e.g., 30 seconds) and determine that the test medium evacuated the testing chamber 404 within the threshold time limit. Accordingly, the controller 422 can determine, in response to the amount of time being less than the threshold, the testing chamber 404 is not blocked.

However, in a second example, the threshold time can be 15 seconds. The controller can compare the amount of time elapsed (e.g., 20 seconds) to the threshold time (e.g., 15 seconds) and determine that the test medium was not evacuated from the testing chamber 404 within the threshold time limit. Accordingly, the controller 422 can determine, in response to the amount of time being greater than the threshold, the testing chamber 404 is blocked.

In some examples, the controller 422 can determine a flow rate of the air that includes the test medium passing through the testing chamber 404. For example, if the flow rate of the air through the testing chamber 404 exceeds a threshold flow rate, the controller 422 can determine that the testing chamber 404 is not blocked. If the flow rate of the air through the testing chamber 404 does not exceed a threshold flow rate, the controller 422 can determine that the testing chamber 404 is blocked.

The above anti-masking testing procedure can be performed according to a particular frequency. For example, the anti-masking testing procedure can be performed every 5 minutes, every 20 minutes, every hour, etc.

During a normal operational mode of the event monitoring and detection device 400, the event monitoring and detection device 400 can be sampling air in the environment surrounding the event monitoring and detection device 400 for real events. The anti-masking testing procedures described above can be performed while the event monitoring and detection device 400 is in the normal operational mode (e.g., not a self-test or maintenance mode). For example, as mentioned above, the controller 422 can cause the self-test module 406 to generate the amount of test medium for the testing chamber 404, where the generated amount of test medium for the testing chamber is less than an alarm threshold of the event monitoring and detection device 400.

Generating the amount of test medium that is less than the alarm threshold for the event monitoring and detection device 400 can allow for the anti-mask test to be completed while the event monitoring and detection device 400 is in a normal operational mode, as the anti-mask test can be performed without causing the event monitoring and detection device 400 to falsely detect an event. However, as the anti-mask testing procedure is being performed, if the event monitoring and detection device 400 does detect an actual event (e.g., smoke generated from a real fire in the facility enters the testing chamber 404 that exceeds the alarm threshold for the event monitoring and detection device 400 while the anti-mask testing procedure is being performed), the event monitoring and detection device 400 can generate and transmit a signal indicating the event monitoring and detection device 400 is in an alarm condition.

Accordingly, event monitoring and detection device testing according to the disclosure can allow for the event monitoring and detection device to measure for particulate matter as well as determine whether the testing chamber of the event monitoring and detection device is blocked utilizing an air movement device included in a self-test module of the event monitoring and detection device. Event monitoring and detection device testing can provide for a more effective, robust, and cost-conscious solution for self-test and particulate monitoring, as compared with previous approaches.

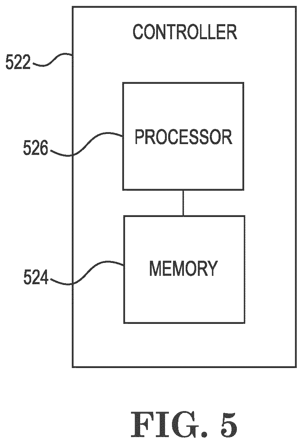

Figure 5 is an example of a controller 522 for event monitoring and detection device testing, in accordance with one or more embodiments of the present disclosure. As illustrated in Figure 5, the controller 522 can include a memory 524 and a processor 526 for event monitoring and detection device testing, in accordance with the present disclosure.

The memory 524 can be any type of storage medium that can be accessed by the processor 526 to perform various examples of the present disclosure. For example, the memory 524 can be a non-transitory computer readable medium having computer readable instructions (e.g., executable instructions/computer program instructions) stored thereon that are executable by the processor 526 for event monitoring and detection device testing in accordance with the present disclosure.

The memory 524 can be volatile or nonvolatile memory. The memory 524 can also be removable (e.g., portable) memory, or non-removable (e.g., internal) memory. For example, the memory 524 can be random access memory (RAM) (e.g., dynamic random access memory (DRAM) and/or phase change random access memory (PCRAM)), read-only memory (ROM) (e.g., electrically erasable programmable read-only memory (EEPROM) and/or compact-disc read-only memory (CD-ROM)), flash memory, a laser disc, a digital versatile disc (DVD) or other optical storage, and/or a magnetic medium such as magnetic cassettes, tapes, or disks, among other types of memory.

Further, although memory 524 is illustrated as being located within controller 522, embodiments of the present disclosure are not so limited. For example, memory 524 can also be located internal to another computing resource (e.g., enabling computer readable instructions to be downloaded over the Internet or another wired or wireless connection).

The processor 526 may be a central processing unit (CPU), a semiconductor-based microprocessor, and/or other hardware devices suitable for retrieval and execution of machine-readable instructions stored in the memory 524.

Although specific embodiments have been illustrated and described herein, those of ordinary skill in the art will appreciate that any arrangement calculated to achieve the same techniques can be substituted for the specific embodiments shown. This disclosure is intended to cover any and all adaptations or variations of various embodiments of the disclosure.

It is to be understood that the above description has been made in an illustrative fashion, and not a restrictive one. Combination of the above embodiments, and other embodiments not specifically described herein will be apparent to those of skill in the art upon reviewing the above description.

The scope of the various embodiments of the disclosure includes any other applications in which the above structures and methods are used. Therefore, the scope of various embodiments of the disclosure should be determined with reference to the appended claims, along with the full range of equivalents to which such claims are entitled.

In the foregoing Detailed Description, various features are grouped together in example embodiments illustrated in the figures for the purpose of streamlining the disclosure. This method of disclosure is not to be interpreted as reflecting an intention that the embodiments of the disclosure require more features than are expressly recited in each claim.

Rather, as the following claims reflect, inventive subject matter lies in less than all features of a single disclosed embodiment. Thus, the following claims are hereby incorporated into the Detailed Description, with each claim standing on its own as a separate embodiment.

## Claims

1. A controller (122, 422, 522) for an event monitoring and detection device (100, 200, 300, 400), comprising:
a memory (124, 524); and
a processor (126, 526) configured to execute executable instructions stored in the memory (124, 524) to:
modify a sensitivity level of a sensor (412) of the event monitoring and detection device (100, 200, 300, 400) from a first sensitivity level to a second sensitivity level;
cause an air movement device (116, 216, 416) to pass air through a testing chamber (104, 204, 404) of the event monitoring and detection device (100, 200, 300, 400);
cause the sensor (412) to take an environmental particulate matter reading of particle sizes of particulates in the air passing through the testing chamber (104, 204, 404); and
transmit the environmental particulate matter reading to a remote computing device (426).

2. The controller of claim 1, wherein the processor is configured to cause a self-test module (106, 406) of the event monitoring and detection device to generate an amount of test medium for the testing chamber.

3. The controller of claim 2, wherein the amount of test medium is less than an alarm threshold.

4. The controller of claim 2, wherein the test medium is an aerosol.

5. The controller of claim 2, wherein the processor is configured to cause the air movement device to evacuate the generated amount of test medium from the testing chamber.

6. The controller of claim 5, wherein the processor is configured to determine whether the testing chamber is blocked based on the air movement device evacuating the test medium from the testing chamber.

7. The controller of claim 1, wherein modifying the sensitivity level of the sensor includes increasing the sensitivity level from the first sensitivity level to the second sensitivity level, wherein the second sensitivity level is more sensitive than the first sensitivity level.

8. The controller of claim 1, wherein the environmental particulate matter reading includes a sample for particulate matter in the air passing through the testing chamber that is smaller than an event threshold size.

9. The controller of claim 1, wherein the processor is configured to modify the sensitivity level of the sensor from the second sensitivity level to the first sensitivity level after causing the environmental particulate matter reading to be taken.

10. The controller of claim 1, wherein the processor is configured to cause the sensor to perform a preliminary event check of smoke in the testing chamber prior to modifying the sensitivity level of the sensor from the first sensitivity level to the second sensitivity level.

11. A method, comprising:
increasing, by a controller (122, 422, 522) of an event monitoring and detection device (100, 200, 300, 400), a sensitivity level of a sensor (412) of the event monitoring and detection device (100, 200, 300, 400) from a first sensitivity level to a second sensitivity level,
causing, by the controller (122, 422, 522), an air movement device (116, 216, 416) of the event monitoring and detection device (100, 200, 300, 400) to pass air through a testing chamber (104, 204, 404) of the event monitoring and detection device (100, 200, 300, 400);
causing, by the controller (122, 422, 522), the sensor to take an environmental particulate matter reading of particle sizes of particulates in the air passing through the testing chamber (104, 204, 404); and
transmitting, by the controller (122, 422, 522), the environmental particulate matter reading to a remote computing device (426).

12. The method of claim 11, wherein the method includes performing an anti-mask test by causing, by the controller, a self-test module (106, 406) of the event monitoring and detection device to generate an amount of test medium for the testing chamber that is less than an alarm threshold for the event monitoring and detection device.

13. The method of claim 12, wherein the method includes evacuating, by the air movement device, the generated amount of test medium from the testing chamber.

14. The method of claim 13, wherein the method includes determining, by the controller, whether the testing chamber is blocked via an obscuration test, wherein the obscuration test includes determining an amount of time for an obscuration level of the sensor to diminish from an obscured value to a start value.

15. The method of claim 13, wherein the method includes determining, by the controller, whether the testing chamber is blocked by determining a flow rate of the air that includes the test medium passing through the testing chamber.
